# EUROPÄISCHE PATENTANMELDUNG

(11) **EP 0 761 175 A1**
(43) Veröffentlichungstag der Anmeldung: **12.03.1997**
(21) Anmeldenummer: 96113434.3
(22) Anmeldetag: 21.08.1996
(51) Int. Cl.: A61B 17/84, F16B 1/00, F16B 13/12

(54) **Schraubbefestigung zur Fixation von Knochenfrakturen**

(30) Priorität: 22.08.1995 AT 9514/14
(71) Anmelder: Enzendorfer, Martin, Dr., 4600 Wels (AT)
(72) Erfinder: Enzendorfer, Martin, Dr., 4600 Wels (AT)
(74) Vertreter: Hiebsch, Gerhard F., Dipl.-Ing.

(57) **Zusammenfassung**

Eine Schraubbefestigung zur Fixation von Knochenfrakturen erfordert eine in ein vorgebohrtes Loch eines Knochens einsetzbare Befestigungsschraube. Um einen ordentlichen Schraubensitz auch bei porösem Knochenmaterial zu gewährleisten, ist der Befestigungsschraube eine ein Muttergewinde (2) aufweisende Ankerhülse (1) zugeordnet, die an ihrem Einsteckende (4) radial auswärts über ihren Außenumfang (10) hinaus vorragende, aber federnd einwärts drückbare Stützpratzen (7) trägt. Diese sind mit auswärts gerichteten widerhakenartigen Klauen (8) versehen.

## Beschreibung

Die Erfindung bezieht sich auf eine Schraubbefestigung zur Fixation von Knochenfrakturen od.dgl. mit einer in ein vorgebohrtes Loch eines Knochens einsetzbaren Befestigungsschraube.

Zur besseren und schnelleren Heilung von Knochenfrakturen ist es in der heutigen Medizin üblich, verletzte Knochen zusammenzuschrauben oder an den Knochen Stützschienen bzw. Stützplatten festzuschrauben, wobei die Befestigungsschrauben in vorgebohrte Löcher des Knochens eingeschraubt werden. Schwierigkeiten bereitet allerdings ein solches Einschrauben der Befestigungsschrauben bei porösem, brüchigem Knochenmaterial; die Schraubengewinde finden keinen Halt und drehen durch, so daß diese Befestigungsschrauben mehr oder weniger lose in den Bohrlöchern stecken und kaum Belastungen aufnehmen können. Die Versuche, solche lose Schrauben nach Aufbohren des Bohrloches durch dickere Schrauben zu ersetzen und damit die gewünschte Verbindung herzustellen, scheitern oft; denn für ein Aufbohren der Bohrlöcher bleibt nur wenig Spielraum, der den porösen Materialbereich nicht zu überbrücken erlaubt. Wegen des operativen Zuganges von einer Seite her ist auch ein Gegenhalt mittels einer Schraubenmutter nicht erreichbar, was immer wieder zu einer unzulänglichen Schraubbefestigung führt.

Der Erfindung liegt daher die Aufgabe zugrunde, diese Mängel zu beseitigen und eine Schraubbefestigung der eingangs geschilderten Art zu schaffen, die ein ordnungsgemäßes Setzen von Befestigungsschrauben auch im porösen Knochenmaterial ermöglicht und auf verhältnismäßig einfache Weise eine ausreichend belastbare Verbindung gewährleistet.

Zur Lösung dieser Aufgabe führt die Lehre des unabhängigen Patentanspruches; die Unteransprüche geben günstige Weiterbildungen an.

Erfindungsgemäß ist der Befestigungsschraube eine ein Muttergewinde aufweisende Ankerhülse zugeordnet, die an ihrem einen Ende -- dem Einsteckende -- radial auswärts über ihren Außenumfang hinaus vorragende, aber federnd einwärts drückbare Stützpratzen trägt. Eine solche Ankerhülse läßt sich auf Grund der einfedernden Stützpratzen ohne weiteres in ein auf den Außenumfang der Ankerhülse abgestimmtes Bohrloch einstecken und durch den Knochen hindurchschieben, bis beim Austreten des Einsteckendes am anderen Ende der Bohrung sich die federnden Stützpratzen aufweiten und an der festen Knochenaußenschicht abstützen. Die in die Ankerhülse einschraubbare Befestigungsschraube findet dadurch über die Ankerhülse und die Stützpratzen am Knochen ausreichend Halt für die gewünschte Befestigung.

Beim Setzen einer Schraubbefestigung wird üblicherweise zuerst ein normales Bohrloch gebohrt und versucht, eine Befestigungsschraube ohne Ankerhülse in den Knochen einzuschrauben. Greift die Schraube ordnungsgemäß, erfolgt die Schraubbefestigung ohne Ankerhülse. Dreht die Schraube durch, wird die Befestigungsschraube aus dem Bohrloch herausgezogen, das Bohrloch aufgeweitet, bis die der Befestigungsschraube zugeordnete Ankerhülse eingeführt werden kann, und dann die Ankerhülse mit Hilfe der vorerst nur teilweise eingeschraubten Befestigungsschraube unter Einfederung der Stützpratzen in das Bohrloch eingeführt. Sobald die Stützpratzen an der beim Operieren nicht zugänglichen gegenüberliegenden Knochenseite verrasten, wird die Befestigungsschraube angezogen und die vorbereitete Befestigung hergestellt. Diese Schraubbefestigungen können zum direkten Verschrauben von Knochenteilen, aber selbstverständlich auch zum Anschrauben von Stützplatten und Schienen, zum Verankern von Implantaten od.dgl. Verwendung finden.

Um nach dem Einsetzen der Ankerhülse beim Eindrehen der Befestigungsschraube ein Mitdrehen der Ankerhülse zu verhindern, können die Stützpratzen widerhakenartige Klauen bilden, und/oder die Ankerhülse kann am Außenumfang axiale Führungsrippen aufweisen. Die Klauen krallen sich schon bei geringen Zugkräften im Knochen fest, so daß eine entsprechende drehfeste Verbindung entsteht, und die Führungsrippen am Außenumfang der Ankerhülse bringen eine über die Hülsenlänge durchgehende Axialführung mit einer gleichzeitigen Drehsicherung mit sich.

An sich könnten die Stützpratzen zum Einfedern beweglich an der Hülse angelenkt sein oder selbst aus federelastischem Material bestehen, doch besonders vorteilhaft ist es, wenn die Ankerhülse vom Einsteckende her -- über vorzugsweise etwa 2/3 der Hülsenlänge -- durch Axialschlitze in Axialzungen aufgeteilt ist, die am freien Ende die Stützpratzen bilden; durch die Axialzungen entstehen entsprechend lange Federzungen, die eine ausreichende Federung mit sich bringen und für den Ansatz der Stützpratzen nur wenig Überstand über das Bohrlochende erfordern, was vor allem aus medizinischen Gründen wichtig ist. Außerdem behindert die Befestigungsschraube nach ihrem Einschrauben das Einfedern der Zungen, wodurch eine Sicherung des Hülsensitzes gewährleistet wird.

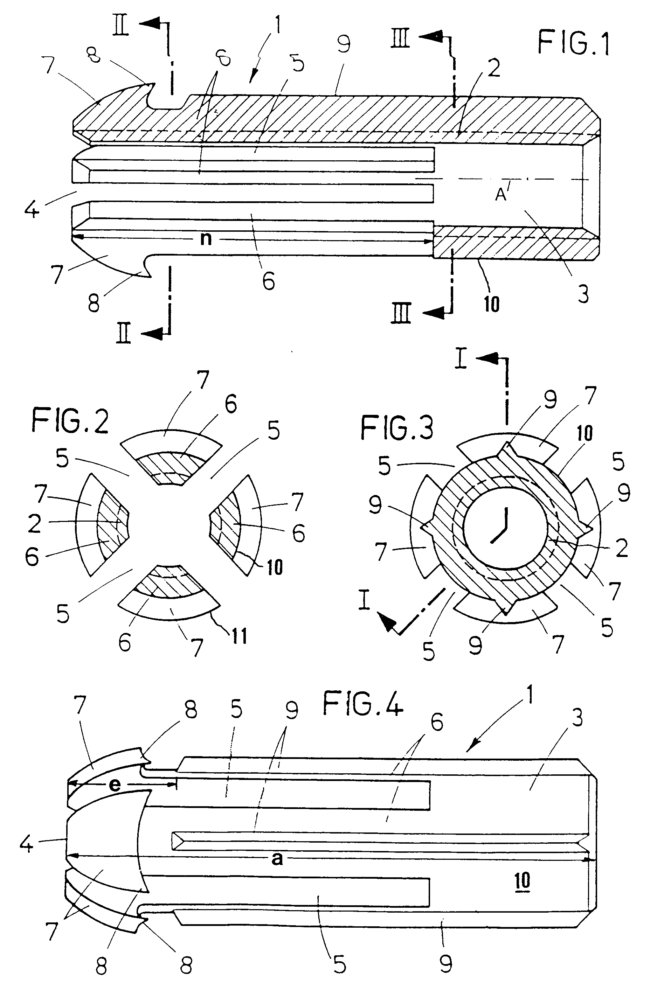

Weitere Vorteile, Merkmale und Einzelheiten der Erfindung ergeben sich aus der nachfolgenden Beschreibung eines bevorzugten Ausführungsbeispiels sowie anhand der Zeichnung; diese zeigt in
- Fig. 1:: einen Längsschnitt durch eine erfindungsgemäße Ankerhülse, die nach Linie I-I der Fig. 3 geschnitten ist;
- Fig. 2 und 3:: Querschnitte nach den Linien II-II bzw. III-III der Fig. 1 und
- Fig. 4:: die Ankerhülse in Seitenansicht.

Eine Ankerhülse 1 für ein zugfestes Setzen einer nicht weiter dargestellten Befestigungsschraube in porösem Knochenmaterial weist ein sich über die Hülsenlänge a erstreckendes Innen- oder Muttergewinde 2 zum Einschrauben der Befestigungsschraube auf und bildet neben einem geschlossenen hinteren Einschraubende 3 ein vorderes Einsteckende 4, von dem aus die Ankerhülse 1 durch zur Längsachse A parallele Axialschlitze 5 der Länge n bis etwa 2/3 der Hülsenlänge a in Axialzungen 6 aufgeteilt ist.

Die Axialzungen 6 weisen an ihrem freien Ende Stützpratzen 7 auf, die widerhakenartige Klauen 8 bilden und in ihrer Grundstellung radial auswärts über den Außenumfang 10 der Ankerhülse 1 vorragen; jene Klauen 8 erzeugen jeweils eine teilkreisförmige Kontur 11. Dank der Zungenausbildung bzw. der durch die Axialschlitze 5 gegebenen Freiräume lassen sich die Stützpratzen 7 aber radial einwärts drücken, bis die Klauen 8 innerhalb des Außenumfanges 10 zu liegen kommen, so daß die Ankerhülse 1 in ein an den Außenumfang 10 angepaßtes -- aus Gründen der Übersichtlichkeit nicht gezeigtes -- Bohrloch eingeschoben werden kann sowie die Stützpratzen 7 erst nach dem Heraustreten aus dem Bohrloch auf der Ausangsseite aufspreizen und ein Rückziehen der Ankerhülse 1 verhindern. Das Einschrauben der Befestigungsschraube in eine solche Ankerhülse 1 führt dann zu einem festen Halt wobei eine Drehsicherung der Ankerhülse 1 innerhalb des Bohrloches durch die Klauen 8 einerseits, aber auch durch axiale Führungsrippen 9 hier dreiecksförmigen Querschnittes am Außenumfang 10 der Ankerhülse 1 anderseits erreicht wird; diese liegen in dem in der Zeichnung dargestellten entspannten Zustand der Ankerhülse 1 innerhalb jener teilkreisförmigen Konturen 11 der Klauen 8.

## Patentansprüche

1. Schraubbefestigung zur Fixation von Knochenfrakturen od.dgl. mit einer in ein vorgebohrtes Loch eines Knochens einsetzbaren Befestigungsschraube,
dadurch gekennzeichnet,
daß der Befestigungsschraube eine ein Muttergewinde (2) aufweisende Ankerhülse (1) zugeordnet ist, die an ihrem einen Ende (4) radial auswärts über ihren Außenumfang (U) hinaus vorragende, federnd einwärts drückbare Stützpratzen (7) trägt.

2. Schraubbefestigung nach Anspruch 1, dadurch gekennzeichnet, daß sich das Muttergewinde (2) über die Hülsenlänge (a) der Ankerhülse (1) erstreckt.

3. Schraubbefestigung nach Anspruch 1 oder 2, dadurch gekennzeichnet, daß das die Stützpratzen (7) tragende Ende das Einsteckende (4) der Ankerhülse (1) bildet.

4. Schraubbefestigung nach einem der Ansprüche 1 bis 3, dadurch gekennzeichnet, daß die Stützpratzen (7) mit auswärts gerichteten widerhakenartigen Klauen (8) versehen sind.

5. Schraubbefestigung nach Anspruch 4, dadurch gekennzeichnet, daß die Klauen (8) jeweils eine teilkreisförmige Kontur (11) bilden.

6. Schraubbefestigung nach einem der Ansprüche 1 bis 5, dadurch gekennzeichnet, daß die Ankerhülse (1) am Außenumfang (10) achsparallele Führungsrippen (9) aufweist.

7. Schraubbefestigung nach Anspruch 5 oder 6, dadurch gekennzeichnet, daß die Führungsrippen (9) bei unbelastetem Zustand der Ankerhülse (1) innerhalb der von den Stützpratzen (7) gebildeten etwa teilkreisförmigen Kontur (11) liegen.

8. Schraubbefestigung nach Anspruch 6 oder 7, gekennzeichnet durch einen dreiecksartigen Querschnitt der in Abstand (e) zum Einsteckende (4) verlaufenden Führungsrippen (9).

9. Schraubbefestigung nach einem der Ansprüche 1 bis 8, dadurch gekennzeichnet, daß die Ankerhülse (1) vom Einsteckende (4) her durch Axialschlitze (5) in Axialzungen (6) aufgeteilt ist, die am Einsteckende die Stützpratzen (7) bilden.

10. Schraubbefestigung nach Anspruch 9, dadurch gekennzeichnet, daß die Länge (n) der Axialschlitze (5) etwa 2/3 der Hülsenlänge beträgt.
